# EUROPÄISCHE PATENTSCHRIFT

(11) **EP 1 600 135 B1**
(45) Veröffentlichungstag und Bekanntmachung des Hinweises auf die Patenterteilung: **17.09.2008**
(21) Anmeldenummer: 05010504.8
(22) Anmeldetag: 13.05.2005
(51) Int. Cl.: A61G 17/007, C12N 1/14, C12N 1/22

(54) **Substrat zur Holzverrottung**
Substrate for wood rotting
Substrat pour la putréfaction du bois

(30) Priorität: 28.05.2004 DE 102004026259
(43) Veröffentlichungstag der Anmeldung: 30.11.2005
(73) Patentinhaber: Schwarze, Francis W.M.R., Dr., 9000 St. Gallen (CH)
(72) Erfinder: Schwarze, Francis W.M.R., Dr., 9000 St. Gallen (CH)
(74) Vertreter: Tetzner, Michael

(56) Entgegenhaltungen:
- DE-A1- 2 128 007
- DE-U- 29 921 202
- US-A- 5 972 672

## Beschreibung

Die Erfindung betrifft die Verwendung eines Substrats zur beschleunigten Holzverrottung, insbesondere ein Substrat zur Sargholzverrottung.

Auf vielen Friedhöfen finden Erdbestattungen seit Jahrhunderten statt. Unter optimalen Standortbedingungen kann damit gerechnet werden, dass die Autolyse des Leichnams und des Sargholzes nach 10 Jahren abgeschlossen ist. Ungünstige Bodenverhältnisse verlängern die Ruhezeiten bei Erdbestattungen erheblich. In circa 35 % der Kommunen Baden-Württembergs können übliche Ruhezeiten (20 - 25 Jahre) nicht eingehalten werden. Eine geringe Luft- und Wasserdurchlässigkeit der Böden, häufig in Verbindung mit Grund-, Hang-, Stau- oder Haftwasser ist dafür verantwortlich, dass ein Standort wenig oder gar nicht für Erdbestattungen geeignet ist. Standorte, an denen die Verrottungsprozesse des Leichnams und des Sarges selbst innerhalb längerer Zeiträume (25 bis mehr als 30 Jahre) nur unzureichend oder überhaupt nicht vollzogen werden, zeichnen sich häufig durch Böden mit einem hohen bis sehr hohen Tongehalt (Pseudogley) und eine hohe Wasserspeicherkapazität aus.

Es gibt empirische Hinweise, dass Friedhofsböden nach Mehrfachbelegung die Tendenz zur Verwesungsmüdigkeit haben können. Ein Grund hierfür dürfte der in der zweiten Hälfte des 20. Jahrhunderts allgemein gestiegene Lebensstandard sein, der sich auch im Anstieg des Marktanteils von hochwertigen Eichensärgen widerspiegelt. Eichenholz besitzt gegenüber Nadelholz eine andere Ligninkomposition sowie einen hohen Gerbstoffgehalt und damit eine deutlich höhere natürliche Dauerhaftigkeit im Boden. Hinzu kommt die höhere Stabilität von Eichensärgen, die ein frühzeitiges Einbrechen des Sargkörpers verhindert. Ein Vordringen der Leicheninsekten in den Zersetzungsraum wird dadurch unterbunden. Deshalb kann bei Bestattungen in Eichen- oder anderen Laubholzsärgen eine Verlängerung der Zersetzungszeit nicht ausgeschlossen werden.

Aus mykologischer Sicht führen auch die zweckorientierte Nutzung der Friedhöfe zu infektionshemmenden Bedingungen, welche die natürlichen Zersetzungsprozesse zusätzlich stark hemmen. Der hohe ästhetische Anspruch an das Erscheinungsbild der Gräber auf Friedhöfen hat neben dem Drang zum täglichen Giessen der Grabbepflanzung zur Folge, dass Totholz und andere organische Substanz (Äste, Streu, Blätter) regelmäßig beseitigt werden. Somit befindet sich in und auf Friedhofsböden häufig eine unzureichende Menge organischer Substanz, die in natürlichen Wäldern das Wachstum und Überdauern von holzzersetzenden Organismen erst ermöglicht. Zwangsläufig wird das Sargholz in Erdgräbern an problematischen Standorten nicht besiedelt und zersetzt, sondern konserviert. Die Beseitigung des Holzes nach Ablauf der Ruhezeit ist aufwendig und muss in Handarbeit erfolgen. Außerdem fallen zusätzliche Kosten für die Entsorgung des Holzes an.

Verschiedene Forschungsansätze bemühen sich vorrangig um eine Beschleunigung der Autolyse des Leichnams. Ziel dieser Überlegungen ist es, die Entstehung von so genannten Wachsleichen zu vermeiden und somit zusätzliche Kosten nach Abschluss der üblichen Ruhezeiten durch eine Feuerbestattung zu vermeiden. Bei diesen Ansätzen kommen vor allem Bakterien zum Einsatz, die aber nicht das Potential besitzen, Holz in großem Umfang abzubauen.

Aus der DE-U1-299 21 202 ist ein Sarg für Bestattungen bekannt, wobei das Sargholz und/oder die Sargausstattung und/oder die Beschläge mit einem Trägermaterial versehen sind, die mit einem Pilzmyzel und/oder Bakterien und/oder sonstigen Holz zerstörenden Organismen geimpft sind, die den Zersetzungsprozess des Sargholzes beschleunigen.

Die US-A-5 972 672 betrifft den Weißfäuleerreger "Phanerochaete chrysosporium", bei dem es sich um eine selektiv ligninabbauende Pilzkultur handelt.

Der Erfindung liegt die Aufgabe zugrunde, die Verwendung eines Substrats anzugeben, welches eine beschleunigte Holzverrottung ermöglicht.

Erfindungsgemäß wird diese Aufgabe durch die Merkmale des Anspruches 1 gelöst.

Weiterhin wird ein Verfahren zur beschleunigten Holzverrottung gemäß dem Anspruch 10 sowie ein Substrat zur Holzverrottung gemäß Anspruch 11 angegeben.

Das erfindungsgemäße Substrat zur Holzverrottung wird durch ein von holzzersetzenden Pilzen durchwachsenes Mineralgestein gebildet, wobei die Wasserkapazität des Mineralgesteins wenigstens 300 Gew.-% beträgt.

Zur beschleunigten Verrottung von Holz wird dieses Substrat mit dem zu verrottenden Holz in unmittelbaren Kontakt gebracht.

Weitere Ausgestaltungen der Erfindung sind Gegenstand der Unteransprüche.

Als Mineralgestein kommt vorzugsweise ein inertes Mineralgestein zum Einsatz, welches insbesondere gepuffert ist und einen pH-Wert in Bereich von 5,5 und 6,5 aufweist.

Bei der Herstellung des Substrats wird das Mineralgestein von den Pilzen durchwachsen.

Weitere Ausgestaltungen und Vorteile der Erfindung werden anhand der folgenden Beschreibung einiger Ausführungsbeispiele näher erläutert.

In der Zeichnung zeigen
- Fig. 1: eine schematische Schnittansicht eines Starter-Kits und
- Fig.2: eine Unteransicht des Starter-Kits

Bei der Herstellung des Substrats wird ein inertes, gepuffertes und wasserspeicherndes Mineralgestein unter kontrollierten Bedingungen mit Pilzmyzelien besiedelt. Dabei kommen zweckmäßigerweise ein oder mehrere verschiedene holzzersetzende Pilze mit unterschiedlichem enzymatischen Potenzial zum Einsatz. Das Mineralgestein wird dabei vor der Beimpfung mit den Myzelien ausgewählter Pilzarten sterilisiert.

Prinzipiell kommen alle holzzersetzenden Pilze für die Herstellung des Substrats in Betracht. Bei den der Erfindung zugrundeliegenden Versuchen hat sich jedoch herausgestellt, dass insbesondere die Pilze aus der Klasse der Hymenomyceten in Betracht kommen. Hierbei haben sich die Pilze aus der Ordnung der Aphyllophorales und/oder Agaricales bzw. die Pilze aus der Familie der Polyporaceae und/oder Tricholomataceae als besonders vorteilhaft herauskristallisiert.

Bei der Anwendung des Substrats zur Holzverrottung wird das Substrat mit dem zu verrottenden Holz in unmittelbaren Kontakt gebracht. Dies kann beispielsweise durch das im folgenden näher beschriebene Starter-Kit erreicht werden.

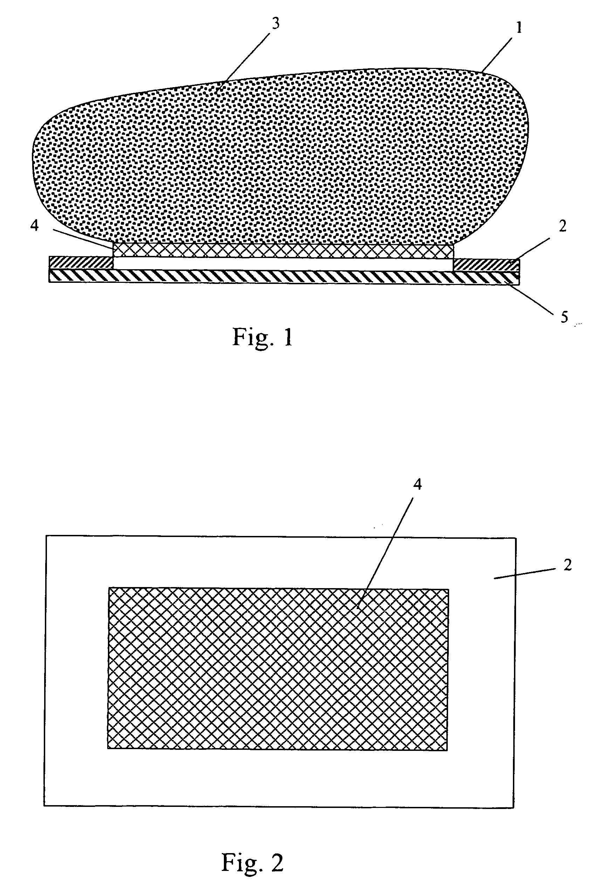

In den Fig. 1 und 2 ist ein Starter-Kit dargestellt, welches unter sterilen Bedingungen abgepacktes, mit Pilzmycelien durchwachsenes Mineralgestein enthält.

Das Starter-Kit besteht aus einer Kunststoffumhüllung 1, die an einem Rahmen 2, insbesondere einem Kartonrahmen befestigt ist. In der Kunststoffumhüllung befindet sich das mit Pilzmycelien durchwachsene Mineralgestein 3 (beispielsweise zwischen 100 - 750 g). Die Unterseite der Kunststoffumhüllung ist im Bereich des Rahmens mit einem feinmaschigen Vlies 4 abgedeckt, um ein Herausrieseln des Produktes zu vermeiden. Der Rahmen 2 weist auf der Unterseite eine Klebstoffschicht auf, die zunächst mit einem Schutzelement 5 abgedeckt ist.

Beim Aufbringen des Starter-Kits wird das Abdeckelement entfernt und der Rahmen mit der Kunststoffumhüllung auf das zu verrottende Holz (beispielsweise einen Sarg) aufgeklebt. Der Pilz kann dann über das Vlies direkt mit dem zu verrottenden Holz in Kontakt kommen.

Die mikroklimatischen Bedingungen innerhalb des Starter-Kits ermöglichen ein Überdauern der holzzersetzenden Pilze, fördern das Wachstum während der Initialinfektion des zu verrottenden Holzes und verringern die Gefahr einer Verunreinigung durch natürliche Gegenspieler.

Das Substrat lässt sich besonders vorteilhaft zur Beschleunigung der Sargholzverrottung einsetzen, wobei das Substrat beispielsweise vor der Bestattung auf dem Boden des Grabes ausgebracht wird und der Sarg nach der Bestattung in unmittelbaren Kontakt mit dem Substrat steht. Alternativ können aber auch ein oder mehrere Starter-Kits am Sarg im Bereich seines Deckels, Bodens und/oder der Seiten angebracht werden, um die Zersetzung zu beschleunigen.

## Patentansprüche

1. Verwendung eines mit Pilzen versehenen Substrats zur beschleunigten Holzverrottung, **dadurch gekennzeichnet, dass** es sich bei dem Substrat um ein von holzzersetzenden Pilzen durchwachsenes Mineralgestein handelt und das Mineralgestein eine Wasserspeicherkapazität von wenigstens 300 Gew.-% aufweist.

2. Verwendung des Substrats nach Anspruch 1, **dadurch gekennzeichnet, dass** es sich um ein inertes Mineralgestein handelt.

3. Verwendung des Substrats nach Anspruch 1, **dadurch gekennzeichnet, dass** es sich um ein gepuffertes Mineralgestein handelt.

4. Verwendung des Substrats nach Anspruch 1, **dadurch gekennzeichnet, dass** das Mineralgestein einen pH-Wert im Bereich von 5,5 und 6,5 aufweist.

5. Verwendung des Substrats nach Anspruch 1, **dadurch gekennzeichnet, dass** die Pilze aus der Klasse der Hymenomyceten stammen.

6. Verwendung des Substrats nach Anspruch 1, **dadurch gekennzeichnet, dass** die Pilze aus der Ordnung der Aphyllophorales und/oder Agaricales stammen.

7. Verwendung des Substrats nach Anspruch 1, **dadurch gekennzeichnet, dass** die Pilze aus der Familie der Polyporaceae und/oder Tricholomataceae stammen.

8. Verwendung des Substrats nach Anspruch 1, **dadurch gekennzeichnet, dass** die Pilze unterschiedliches enzymatisches Potential aufweisen.

9. Verwendung des Substrats nach Anspruch 1, **dadurch gekennzeichnet, dass** das Mineralgestein mit wenigstens zwei verschiedenen Pilzarten durchwachsen ist.

10. Verfahren zur Beschleunigung der Holzverrottung, **dadurch gekennzeichnet, dass** ein Substrat nach einem oder mehreren der Ansprüche 1 bis 9 verwendet und mit dem zu verrottenden Holz in unmittelbaren Kontakt gebracht wird.

11. Substrat zur Holzverrottung, **gekennzeichnet durch** ein von holzzersetzenden Pilzen durchwachsenes Mineralgestein, wobei das Mineralgestein eine Wasserspeicherkapazität von wenigstens 300 Gew.-% aufweist.

## Claims

1. Use of a substrate, provided with fungi, for accelerated wood rotting, **characterised in that** the substrate is a mineral rock through which wood-decomposing fungi have grown, and the mineral rock has a water-storage capacity of at least 300 wt.%.

2. Use of the substrate according to claim 1, **characterised in that** the substrate is an inert mineral rock.

3. Use of the substrate according to claim 1, **characterised in that** the substrate is a buffered mineral rock.

4. Use of the substrate according to claim 1, **characterised in that** the mineral rock has a pH in the region of 5.5 and 6.5.

5. Use of the substrate according to claim 1, **characterised in that** the fungi belong to the class *Hymenomycetes*.

6. Use of the substrate according to claim 1, **characterised in that** the fungi belong to the order *Aphyllophorales* and/or *Agaricales*.

7. Use of the substrate according to claim 1, **characterised in that** the fungi belong to the family *Polyporaceae* and/or *Tricholomataceae*.

8. Use of the substrate according to claim 1, **characterised in that** the fungi have different enzymatic potentials.

9. Use of the substrate according to claim 1, **characterised in that** at least two different types of fungus have grown through the mineral rock.

10. A method of accelerating wood rotting, **characterised in that** a substrate according to one or more of claims 1 to 9 is used and is brought into direct contact with the wood to be rotted.

11. A substrate for wood rotting, **characterised by** a mineral rock through which wood-decomposing fungi have grown, wherein the mineral rock has a water-storage capacity of at least 300 wt.%.

## Revendications

1. Utilisation d'un substrat pourvu de champignons pour accélérer la décomposition du bois, **caractérisée en ce que** le substrat est une roche minérale entrelardée de champignons décomposant le bois, et **en ce que** la roche minérale présente une capacité de rétention d'eau d'au moins 300 % en poids.

2. Utilisation du substrat selon la revendication 1, **caractérisée en ce qu'**il s'agit d'une roche minérale inerte.

3. Utilisation du substrat selon la revendication 1, **caractérisée en ce qu'**il s'agit d'une roche minérale

4. Utilisation du substrat selon la revendication 1, **caractérisée en ce que** la roche minérale présente une valeur de pH située dans le domaine de 5,5 et 6,5.

5. Utilisation du substrat selon la revendication 1, **caractérisée en ce que** les champignons appartiennent à la classe des hyménomycètes.

6. Utilisation du substrat selon la revendication 1, **caractérisée en ce que** les champignons appartiennent à l'ordre des aphyllophoracés et/ou des agaricacés.

7. Utilisation du substrat selon la revendication 1, **caractérisée en ce que** les champignons appartiennent à la famille des polyporacés et/ou des tricholomatacés.

8. Utilisation du substrat selon la revendication 1, **caractérisée en ce que** les champignons présentent un potentiel enzymatique différent.

9. Utilisation du substrat selon la revendication 1, **caractérisée en ce que** la roche minérale est entrelardée d'au moins deux genres de champignons.

10. Procédé pour accélérer la décomposition du bois, **caractérisé en ce qu'**un substrat selon l'une ou plusieurs des revendications 1 à 9 est utilisé et mis en contact direct avec le bois à décomposer.

11. Substrat pour la décomposition du bois, **caractérisé par** une roche minérale entrelardée de champignons décomposant le bois, la roche minérale présentant une capacité de rétention d'eau d'au moins 300 % en poids.
